# EUROPEAN PATENT SPECIFICATION

(11) **EP 1 747 761 B1**
(45) Date of publication and mention of the grant of the patent: **14.10.2009**
(21) Application number: 05016399.7
(22) Date of filing: 28.07.2005
(51) Int. Cl.: A61B 18/14

(54) **An electrode assembly with electrode cooling element for an electrosurgical instrument**
Elektrodenanordnung mit Elektrodenkühlkörper für ein elektrochirurgisches Gerät
Ensemble électrode comportant un élément de refroidissement d'électrode pour un instrument électrochirurgical

(43) Date of publication of application: 31.01.2007
(73) Proprietor: Covidien AG, 8212 Neuhausen am Rheinfall (CH)
(72) Inventor: Chapman, Troy J., Englewood, Colorado 80112 (US); Shields, Chelsea, Portland, Oregon 97219 (US); Schechter, David A., Longmont, Colorado 80503 (US); Hammill, Curt D., Erie, CO 80516 (US); Podhajsky, Ronald J., Boulder, CO 80301 (US)
(74) Representative: HOFFMANN EITLE

(56) References cited:
- US-A1- 2002 115 997
- US-A1- 2003 097 130
- US-A1- 2003 216 732
- US-A1- 2004 030 332

## Description

### BACKGROUND

### Technical Field

The present disclosure relates to electrosurgical instruments used for open and endoscopic surgical procedures for sealing or fusing tissue. More particularly, the present disclosure relates to a bipolar forceps for sealing vessels, vascular tissues and soft tissues having an electrode sealing assembly which is designed to limit and/or reduce by rapid cooling thermal spread to adjacent tissue structures. For a disclosure of such an instrument, see US 2002/0115997, which serves as a basis for the pre-characterising part of claim 1 below.

### Related Prior Art

Electrosurgical forceps utilize both mechanical clamping action and electrical energy to effect hemostasis by heating the tissue and blood vessels to coagulate and/or cauterize vessels or tissue. However, certain surgical procedures may require sealing blood vessels or vascular tissue rather than just simply effecting hemostasis. "Vessel sealing" or "Tissue Fusion" is defined as the process of liquefying the collagen, elastin and ground substances in the tissue so that it reforms into a fused mass with significantly-reduced demarcation between the opposing tissue structures. In contrast, the term "cauterization" is defined as the use of heat to destroy tissue (also called "diathermy" or "electrodiathermy") and the term "coagulation" is defined as a process of desiccating tissue wherein the tissue cells are ruptured and dried. Coagulation of small vessels is usually sufficient to permanently close them. Larger vessels or tissue need to be "sealed" to assure permanent closure.

Numerous electrosurgical instruments have been proposed in the past for various open and endoscopic surgical procedures. However, most of these instruments cauterize or coagulate tissue and are normally not designed to provide uniformly reproducible pressure on the blood vessel or tissue which, if used for sealing purposes, would result in an ineffective or non-uniform seal. For example, U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stern et al., U.S. Patent No. 5,484,436 to Eggers et al. and U.S. Patent No. 5,951,549 to Richardson et al., all relate to electrosurgical instruments for coagulating, cauterizing, and cutting vessels or tissue.

Many of these instruments include blade members or shearing members which simply cut tissue in a mechanical and/or electromechanical manner and are relatively ineffective for vessel sealing purposes. Other instruments generally rely on clamping pressure alone to procure proper sealing thickness and are often not designed to take into account gap tolerances and/or parallelism and flatness requirements which are parameters which, if properly controlled, can assure a consistent and effective tissue seal. For example, it is known that it is difficult to adequately control thickness of the resulting sealed tissue by controlling clamping pressure alone for either of two reasons: 1) if too much force is applied, there is a possibility that the two poles will touch and energy will not be transferred through the tissue resulting in an ineffective seal; or 2) if too low a force is applied, a thicker less reliable seal is created.

Commonly-owned U.S. Application Serial Nos. PCT Application Serial No. PCT/US01/11340 filed on April 6, 2001 by Dycus, et al. entitled "VESSEL SEALER AND DIVIDER", U.S. Application Serial No. 10/116,824 filed on April 5, 2002 by Tetzlaff et al. entitled "VESSEL SEALING INSTRUMENT" and PCT Application Serial No. PCT/US01/11420 filed on April 6, 2001 by Tetzlaff et al. entitled "VESSEL SEALING INSTRUMENT" teach that to effectively seal tissue or vessels, especially large vessels, two predominant mechanical parameters must be accurately controlled: 1) the pressure applied to the vessel; and 2) the gap distance between the conductive tissue contacting surfaces (electrodes). As can be appreciated, both of these parameters are affected by the thickness of the vessel or tissue being sealed. Accurate application of pressure is important for several reasons: to reduce the tissue impedance to a low enough value that allows enough electrosurgical energy through the tissue; to overcome the forces of expansion during tissue heating; and to contribute to the end tissue thickness which is an indication of a good seal.

It has been found that using electrosurgical instruments to seal tissue may result in some degree of so-called "thermal spread" across adjacent tissue structures. "Thermal spread" refers generally to the heat transfer traveling along the periphery of the electrically conductive surfaces. This can also be termed "collateral damage" to adjacent tissue. As can be appreciated, reducing the thermal spread during an electrical procedure reduces the likelihood of unintentional or undesirable collateral damage to surrounding tissue structures which are adjacent to an intended treatment site. Reducing the collateral damage to surrounding tissue or maintaining the viability of surrounding tissue after the sealing process is known to promote tissue healing and decrease overall healing time by stimulating / improving healing response. Controlling tissue cooling may also reduce adhesion or buildup of tissue on the electrodes and also assist during the formation of the tissue seal, e.g., cross-linking or other chemical bonding, during the reformation or renaturation of collagen.

Instruments which include dielectric coatings disposed on the outer surfaces are known and are used to prevent tissue "blanching" at points normal to the sealing site. In other words, these coatings are primarily designed to reduce accidental burning of tissue as a result of incidental contact with the outer surfaces of the end effectors. So far as is known, these coatings are not designed or intended to reduce collateral tissue damage or thermal spread to adjacent tissue (tissue lying along the tissue plane).

Commonly-owned U.S. Patent Serial No. 10/474,168 entitled "ELECTROSURGICAL INSTRUMENT WHICH REDUCES COLLATERAL DAMAGE TO ADJACENT TISSUE" filed on October 3, 2003 by Buysse et al. relates to an instrument which is configured to control or regulate the electrical field around the electrically conductive sealing surfaces to reduce stray current concentrations which can result in thermal spread to adjacent tissue structures.

Thus, a need exists to develop an electrosurgical instrument which includes an electrode sealing assembly which can seal vessels and tissue consistently and effectively and reduce the undesirable effects of thermal spread across or to adjacent tissue structures by utilizing a thermally conductive, electrically non-conductive material.

In addition, in tissue fusion applications that utilize energy to treat tissue, the need exists to maximize and enhance tissue strength at the tissue fusion site and minimize detrimental tissue effects to adjacent or surrounding tissue structures.

### SUMMARY

It is an object of the present disclosure to provide an electrode sealing assembly designed for use with an electrosurgical instrument for sealing tissue which rapidly cools during or after tissue fusion heating processes.

The invention is defined in claim 1 below and the dependent claims are directed to optimal or preferred features.

The present disclosure generally relates to an electrode sealing assembly for use with an electrosurgical instrument for sealing tissue. The electrode sealing assembly includes first and second jaw members which are movable from a first position in spaced relation relative to one another to at least one second position for grasping tissue therebetween. The jaw members include electrically conductive sealing plates disposed in opposing relation to one another. At least one jaw member includes a thermoelectric cooling plate having a first surface in direct contact with an outer surface of the sealing plate. The thermoelectric cooling plate include first and second electrical connections disposed on opposite sides of the thermoelectric cooling plate. The first connection is configured to selectively transmit a first electrical potential and the second connection is configured to selectively transmit a second electrical potential such that heat generated by the sealing plates is transferred away from the tissue via the thermoelectric cooling plate.

The heat sink is configured to be coupled to an ultimate heat sink for transferring heat from the jaw member(s). The heat sink may include a coolant line disposed therethrough. The coolant line may be configured to receive a coolant to transfer heat from the thermoelectric cooling plate. In one embodiment, the coolant is a thermally conductive, non-electrically conductive fluid which may be one of the group consisting of air, nitrogen, carbon dioxide, and 3M™ Fluorinert™ Electronic Liquid FC-7 (available from 3M Company, St. Paul, Minnesota).

In one particularly useful embodiment, the present disclosure relates to an electrode sealing assembly designed for use with an electrosurgical instrument for sealing tissue. The electrode sealing assembly includes first and second electrode jaw members which are movable from a first position in spaced relation relative to one another to at least one second position for grasping tissue therebetween. The jaw members include sealing plates disposed in opposing relation relative to one another. Each jaw member includes a cooling line disposed therethrough which is configured to convey a cooling liquid therethrough to absorb heat from the sealing plates during or after sealing.

The cooling line may be configured to be coupled to a second or an ultimate heat sink for transferring heat from the jaw member(s). In addition, the coolant line may be configured to receive a coolant to transfer heat from the jaw member(s). In one embodiment, the coolant is a thermally conductive, non-electrically conductive fluid.

### BRIEF DESCRIPTION OF THE DRAWINGS

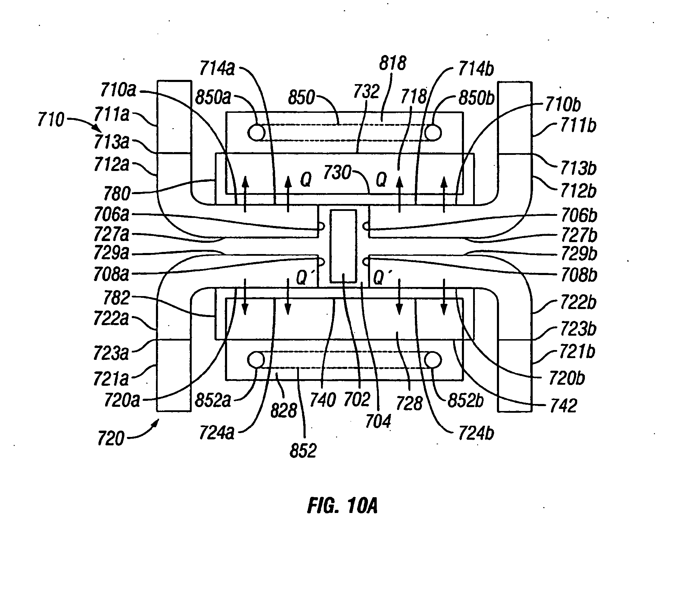

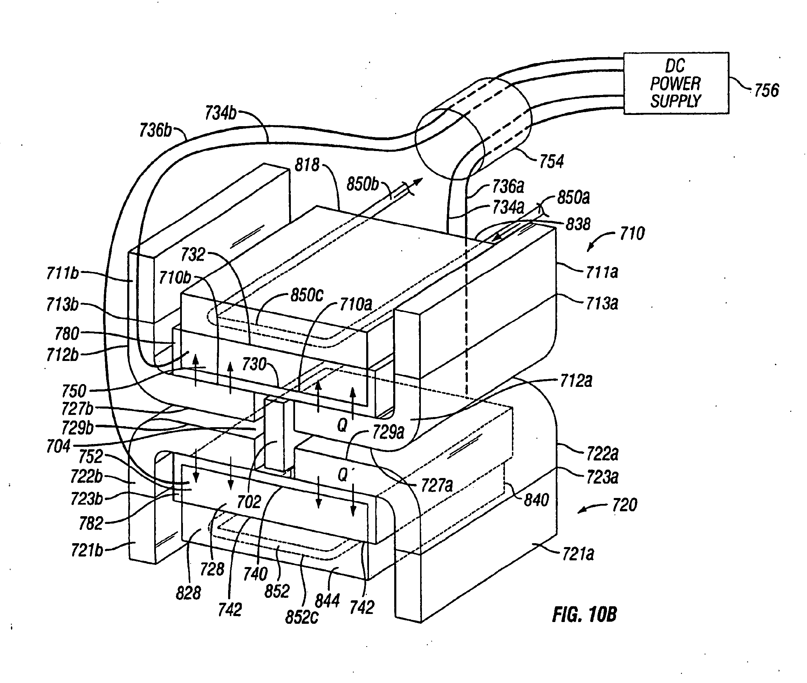

Various instruments are described herein with reference to the drawings of which only Fig. 10A and 10B show an electrode sealing assembly according to the present invention.
FIG. 1A is a perspective view of an endoscopic bipolar forceps which is configured to support an electrode sealing assembly ;
FIG. 1B is a perspective view of an open bipolar forceps which is configured to support the electrode sealing assembly;
FIG. 2A is an enlarged, perspective view of the electrode sealing assembly;
FIG. 2B is an enlarged, perspective view of the embodiment shown in FIG. 2A with parts separated;
FIG. 3 is an enlarged, perspective view of an alternate, simplified embodiment of the electrode sealing assembly with parts separated ;
FIG. 4 is an enlarged, perspective view of an alternate embodiment of the electrode sealing assembly showing an active cooling system designed to reduce thermal spread during activation;
FIG. 5A is an enlarged view of a seal utilizing a conventional vessel sealing instrument with a conventional electrode sealing assembly;
FIG. 5B is an enlarged view of a seal utilizing a vessel sealing instrument having the electrode sealing assembly;
FIG. 6 is a schematic, end view of an alternate electrode sealing assembly which may be utilized to reduce thermal spread during activation;
FIG. 7 is a schematic, end view of another alternate electrode sealing assembly which may be utilized to reduce thermal spread during activation;
FIG. 8A shows a perspective view of a sealed tissue area of an end-to-end anastomosis utilizing a straight electrode sealing assembly;
FIG. 8B shows a perspective view of a sealed tissue area of an end-to-end anastomosis utilizing a curved electrode sealing assembly;
FIG. 9A shows an end view of the jaw members of an electrode sealing assembly which are configured to support an electrode cooling assembly;
FIG. 9B shows a perspective view of the jaw members according to FIG. 9A;
FIG. 9C shows a top perspective view of the jaw members of an electrode sealing assembly which are configured to support another electrode cooling assembly;
FIG. 9D shows a bottom perspective view of the jaw members according to FIG. 9C.
FIG. 10A shows an end view of jaw members of an electrode sealing assembly according to the present invention which are configured to support an electrode cooling assembly ;
FIG. 10B shows a perspective view of the jaw members according to FIG. 10A;
FIG. 11 shows a perspective view of the jaw members of an electrode sealing assembly which are configured to support yet another electrode cooling assembly ;
FIG. 12 is an enlarged, perspective view of yet another alternate embodiment of the electrode sealing assembly of FIG. 4 showing an active cooling system designed to reduce thermal spread during activation;
FIG. 13A is a cross-sectional end view of an embodiment of a cooling line for an electrode cooling assembly;
FIG. 13B is a cross-sectional end view of an alternate embodiment of a cooling line for an electrode cooling assembly;
FIG. 14A is a perspective view of the endoscopic bipolar forceps of FIG. 1A which is configured to support the cooling lines of FIG. 4, FIG. 10A, FIG. 10B, FIG. 11, and FIG. 12; and
FIG. 14B is a perspective view of the open bipolar forceps of FIG. 1B which is configured to support the cooling lines of FIG. 4, FIG. 10A, FIG. 10B. FIG. 11. and FIG. 12.

Claim 1 defines a thermoelectric cooling plate with a first surface and a sealing plate with an outer surface, relative to the inner surface tissue contacting surface, where a thermally conductive, electrically insulating material is disposed therebetween. Claim 1 further defines a heat sink having a coolant line disposed therethrough. These features are shown in Figure 10, which thus shows an embodiment of the present invention.

### DETAILED DESCRIPTION

It has been found that by providing a thermally conductive and electrically non-conductive material adjacent to the electrically conductive sealing surfaces, surgeons can more readily and more easily produce a consistent, high quality seal and effectively reduce thermal spread across or to adjacent tissue. For the purposes herein the term "thermal spread" refers generally to the heat transfer (heat conduction, heat convection or electrical current dissipation) dissipating along the periphery of the electrically conductive or electrically active surfaces to adjacent tissue. This can also be termed "collateral damage" to adjacent tissue and is further discussed in WO-A-2004/098383 entitled "ELECTROSURGICAL INSTRUMENT WHICH REDUCES THERMAL DAMAGE TO ADJACENT TISSUE".

It is envisioned that the configuration of the thermally conductive material which surrounds the perimeter of the electrically conductive surface will effectively absorb heat during electrosurgical activation (or thermally dissipate the heat during electrosurgical activation) and generally restrict heat travel to areas between the opposing electrically conductive surfaces. In other words, the material acts like a so called "heat sinks". As mentioned above, the thermally conductive material is also electrically non-conductive which also restricts current concentrations to between the two opposing surfaces.

It is important to note that this is different from dielectrically coating the outer surfaces of the instrument to prevent tissue "blanching" at points normal to the sealing site. These coatings are not designed or intended to reduce collateral tissue damage or thermal spread to adjacent tissue (tissue lying along the tissue sealing plane).

It is contemplated that by providing a thermally conductive material adjacent to the electrically conductive surface, the thermally conductive path is altered thereby influencing the thermal spread/collateral damage to adjacent tissue structures. In addition, the thermally conductive, electrically non-conductive material also isolates the two electrically opposing poles (i.e., electrodes) from one another thereby reducing the possibility that tissue or tissue fluids can create an unintended bridge or path for current travel to adjacent tissue. The thermally conductive material and electrically conductive sealing surface may be dimensioned such that the current is concentrated at the intended sealing site between the opposing electrically conductive surfaces as explained in more detail below.

It is contemplated that by providing additional cooling of the electrosurgical jaw members of the bipolar forceps such as by solid state cooling via thermoelectric coolers (TEC) based on the Peltier effect, the thermal spread/collateral damage to adjacent tissue structures may also be further reduced. It is further contemplated that additional cooling may be provided to the electrosurgical jaw members via a cooling duct passing internally through the jaw members.

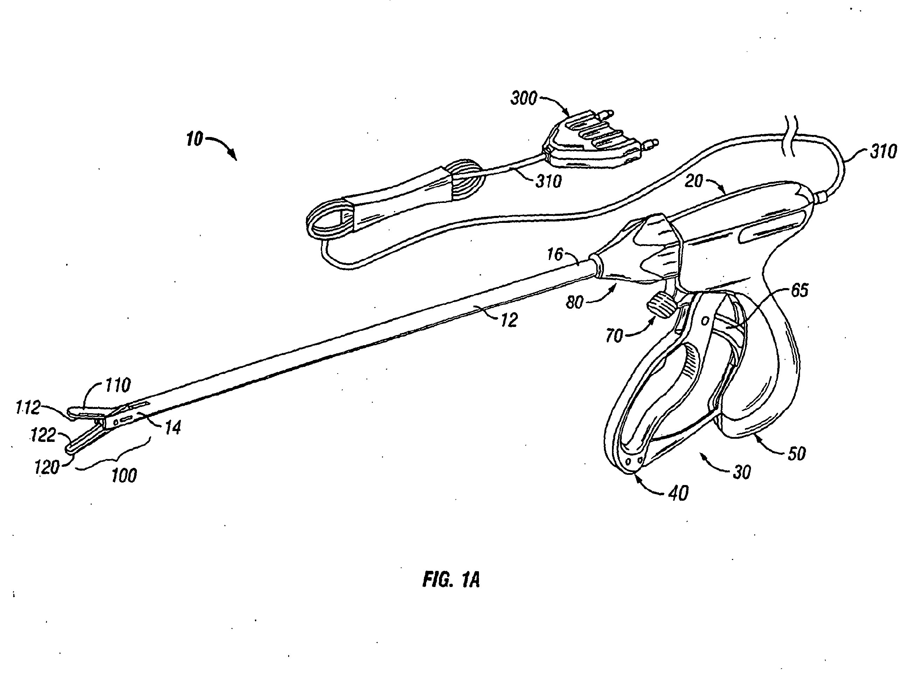

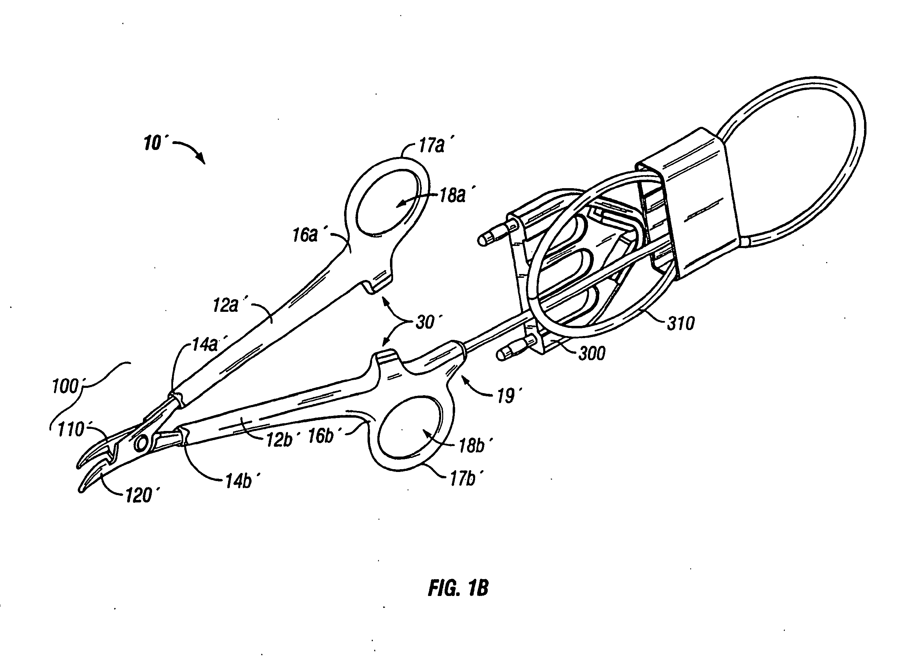

Referring now to FIGS. 1A and 1B, two bipolar forceps 10 and 10' are shown; a first forceps 10 for use with endoscopic surgical procedures and a second forceps 10' for use with open surgical procedures. For the purposes herein, either an endoscopic instrument or an open instrument may be utilized for supporting the electrode sealing assembly . Obviously, different electrical and mechanical connections and considerations apply to each particular type of instrument, however, the novel aspects with respect to the electrode sealing assembly and its operating characteristics remain generally consistent with respect to both the open or endoscopic designs of FIGS. 1A and 1B. Forceps 10 and 10' are shown by way of example and other electrosurgical forceps are also envisioned which may support the electrode sealing assembly of the present disclosure. In the drawings and in the description which follows, the term "proximal", as is traditional, will refer to the end of the forceps 10, 10' which is closer to the user, while the term "distal" will refer to the end which is further from the user.

FIG. 1A shows one example of an endoscopic vessel sealing instrument 10 which is configured to support an electrode sealing assembly 100. More particularly, forceps 10 generally includes a housing 20, a handle assembly 30, a rotating assembly 80, a trigger assembly 70 and the end effector assembly 100 which mutually cooperate to grasp, seal and, if warranted, divide tissue. The forceps 10 includes a shaft 12 which has a distal end 14 dimensioned to mechanically engage the end effector assembly 100 and a proximal end 16 which mechanically engages the housing 20 proximate the rotating assembly 80.

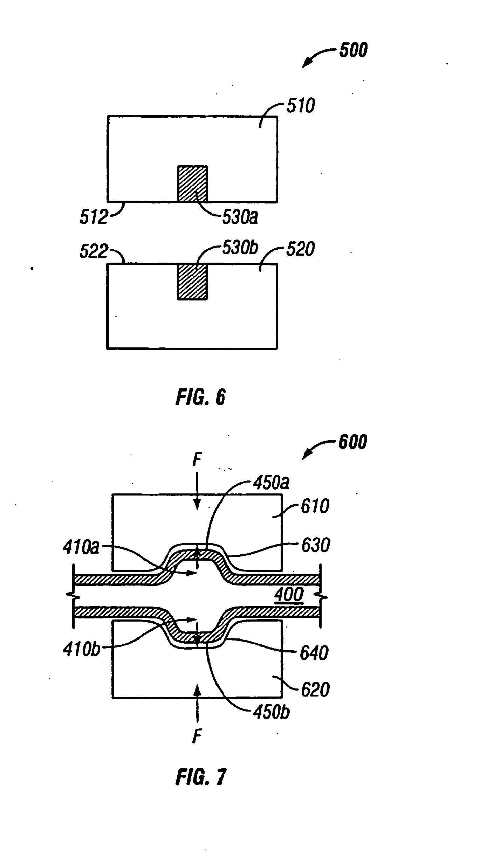

Forceps 10 also includes a plug 300 which connects the forceps 10 to a source of electrosurgical energy, e.g., an electrosurgical generator (not shown) via an electrical cable 310. Handle assembly 30 includes a fixed handle 50 and a movable handle 40. Handle 40 moves relative to fixed handle 50 to actuate the end effector assembly 100 and enable a user to grasp and manipulate tissue 400 (See FIG. 6). More particularly, the end effector assembly 100 includes a pair of opposing jaw members 110 and 120 which move in response to movement of the handle 40 from an open position wherein the jaw members 110 and 120 are disposed in spaced relation relative to one another, to a clamping or closed position wherein the jaw members 110 and 120 cooperate to grasp tissue therebetween.

The housing 20 encloses a drive assembly (not shown) which cooperates with the movable handle 40 to impart movement of the jaw members 110 and 120 from the open position to the clamping or closed position. The handle assembly 30 can generally be characterized as a four-bar mechanical linkage which provides a unique mechanical advantage when sealing tissue between the jaw members 110 and 120. For example, once the desired position for the sealing site is determined and the jaw members 110 and 120 are properly positioned, handle 40 may be compressed fully to lock the jaw members 110 and 120 in a closed position against the tissue. The details relating to the inter-cooperative relationships of the inner-working components of forceps 10 are disclosed in commonly-owned U.S. Patent Application Serial No. 10/284,562 and U.S. Patent Application Serial No. 10/460,926 When the jaw members 110 and 120 are fully compressed about the tissue, the forceps 10 is now ready for selective application of electrosurgical energy.

Experimental results suggest that the magnitude of pressure exerted on the tissue by the electrically conductive sealing surfaces 112, 122 of the jaw members 110 and 120, respectively, is important in assuring a proper surgical seal. Pressures within a working range of about 3 kg/cm² to about 16 kg/cm² and, preferably, within a working range of about 6 kg/cm² to about 13 kg/cm² have been shown to be effective for sealing various tissue types. Most preferably, the pressures are within a working range of about 4.5 kg/cm² to about 8.5 kg/cm² to optimize sealing.

An open forceps 10' for use in connection with traditional open surgical procedures and is shown by way of example in FIG. 1B. Open forceps 10' includes a pair of elongated shaft portions 12a', 12b' each having a proximal end 16a' and 16b', respectively, and a distal end 14a' and 14b', respectively. The forceps 10' includes jaw assembly 100' which attaches to the distal ends 14a' and 14b' of shafts 12a' and 12b', respectively. Jaw assembly 100' includes an upper jaw member 110' and a lower jaw member 120' which are movable relative to one another to grasp tissue therebetween.

Each shaft 12a' and 12b' may include a handle 17a' and 17b' disposed at the proximal end 16a' and 16b' thereof which each define a finger hole 18a' and 18b', respectively, therethrough for receiving a finger of the user. As can be appreciated, finger holes 18a' and 18b' facilitate movement of the shafts 12a' and 12b' relative to one another which, in turn, pivot the jaw members 110' and 120' from the open position wherein the jaw members 110' and 120' are disposed in spaced relation relative to one another for manipulating tissue to a clamping or closed position wherein the jaw members 110' and 120' cooperate to grasp tissue therebetween.

A ratchet 30' is included for selectively locking the jaw members 110' and 120' relative to one another at various positions during pivoting. Preferably, each position associated with the cooperating ratchet interfaces 30' holds a specific, i.e., constant, strain energy in the shaft members 12a' and 12b' which, in turn, transmits a specific closing force to the jaw members 110' and 120'. It is envisioned that the ratchet 30' may include graduations or other visual markings which enable the user to easily and quickly ascertain and control the amount of closure force desired between the jaw members 110' and 120'. One of the shafts, e.g., 12b', includes a proximal shaft connector /flange 19' which is designed to connect the forceps 10' to a source of RF energy (not shown) via an electrosurgical cable 310 and plug 300. The details relating to the inner-working electrical connections and various components of forceps 10' are disclosed in commonly-owned U.S. Patent Application No. 10/369,894

As mentioned above, two mechanical factors play an important role in determining the resulting thickness of the sealed tissue and effectiveness of the seal, i.e., the pressure applied between opposing jaw members 110' and 120' and the gap between the opposing jaw members 110' and 120' during the sealing process. Applying the correct force is also important for other reasons: to reduce the impedance of the tissue to a low enough value that allows enough current through the tissue; and to overcome the forces of expansion during the heating of the tissue in addition to contributing towards creating the required seal thickness necessary for a good seal.

For the purposes herein, electrode assemblies 100 and 100' include the same general configuration and are designed to reduce thermal spread to adjacent tissue. However, certain modifications may have to be made to each electrode sealing assembly 100 (or 100') to fit the electrode sealing assembly 100 (or 100') to a specific support structure for an open or endoscopic instrument. By controlling the intensity, frequency and duration of the RF energy applied to the tissue, the user can selectively seal the tissue as needed for a particular purpose. As can be appreciated, different tissue types and the physical characteristics associated with each tissue type may require different electrical sealing parameters.

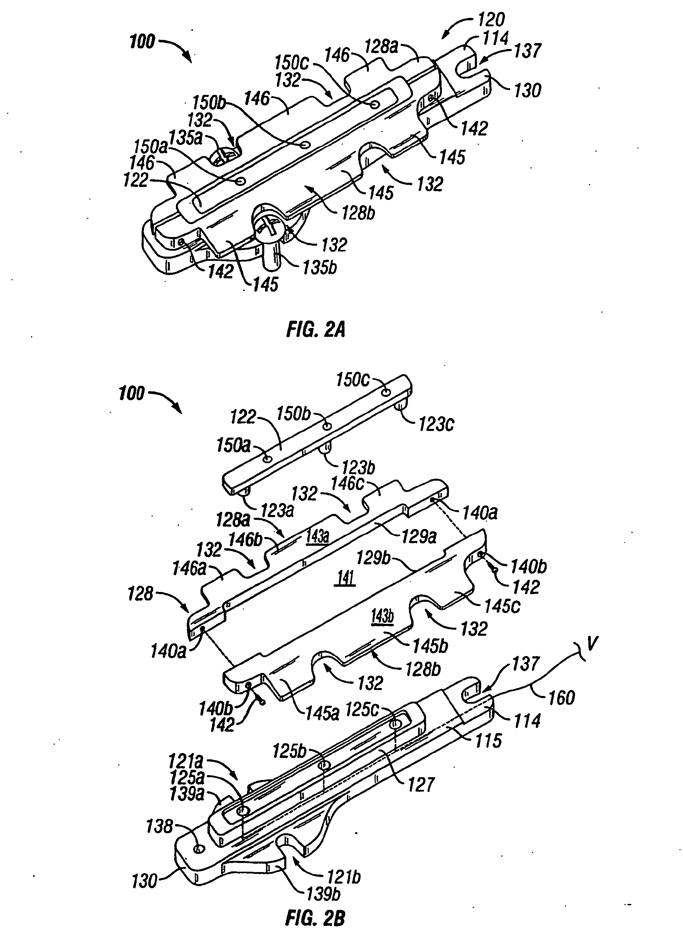

FIGS. 2A and 2B show enlarged views of the lower jaw 120 of the electrode sealing assembly 100 (or 100') according to the present disclosure. As can be appreciated a second jaw 110 with similar components as described below is positioned in opposition to jaw member 120. Only the elements of jaw member 120 are described herein, however, jaw member 110 also includes identical or similar elements which are designed to accomplish similar purposes such that bipolar electrosurgical energy can be conducted through tissue held between the two jaw members 110 and 120 to effect a seal.

More particularly, lower jaw member 120 includes an insulated outer housing 114 which supports a thermally conductive, electrically non-conductive material 128 and electrically conductive sealing surface or sealing plate 122. As best seen in FIG. 2B, insulating housing 114 includes a support surface 115 which houses an electrode support step 127. Support step 127 includes a series of electro-mechanical interfaces 125a, 125b and 125c which matingly engage a set of corresponding interfaces 123a, 123b and 123c which depend from sealing plate 122. The outer periphery of the support step 127 is also preferably dimensioned to matingly engage the thermally conductive material 128 as will be explained in more detail below.

Each electromechanical interface, e.g., 125a, is electrically connected to an electrical potential by way of wire 160 which extends to the generator (not shown). It is envisioned that other electrical configurations are plausible as is known in the art and the above is shown by way of example. For example, electrically conductive tubes or plates may be utilized within the jaw members 110 and 120 to supply current to the sealing plate 122.

Support surface 115 also includes a series of notches 137, 121 a, 121 b and screw holes 138 which secure the insulating housing 114 to the electrode sealing assembly 100. For example, and as best shown in FIG. 2A, the support surface 115 includes a pair of flanges 139a and 139b which project laterally from the distal end of the support surface 115 and which are each dimensioned to receive the head of a screw 135a and 135b, respectively. In turn, the screws 135a and 135b secure the support surface to the electrode sealing assembly 100. A proximal notch 137 mates with another screw (not shown) to position the end of the support surface 115 on the electrode sealing assembly 100. Other apertures, e.g., 138, may also be utilized to align and/or secure the support surface 115 on the electrode sealing assembly 100 during the manufacturing process.

Thermally conductive material 128 is may be made from two laterally-opposing segments 128a and 128b which mate to encompass the sealing plate 122 and the support step 127 as best seen in FIG. 2A. A series of set screws or pegs 142 secure the two thermally conductive segments 128a and 128b about the sealing plate 122 and about the support step 127 once assembled. As mentioned above, the thermally conductive material 128 is designed to effectively absorb or thermally dissipate the heat during electrosurgical activation and generally restrict heat travel to areas between the opposing sealing plates 122. In other words, the material acts like a "heat sink" to limit thermal damage to surrounding tissue.

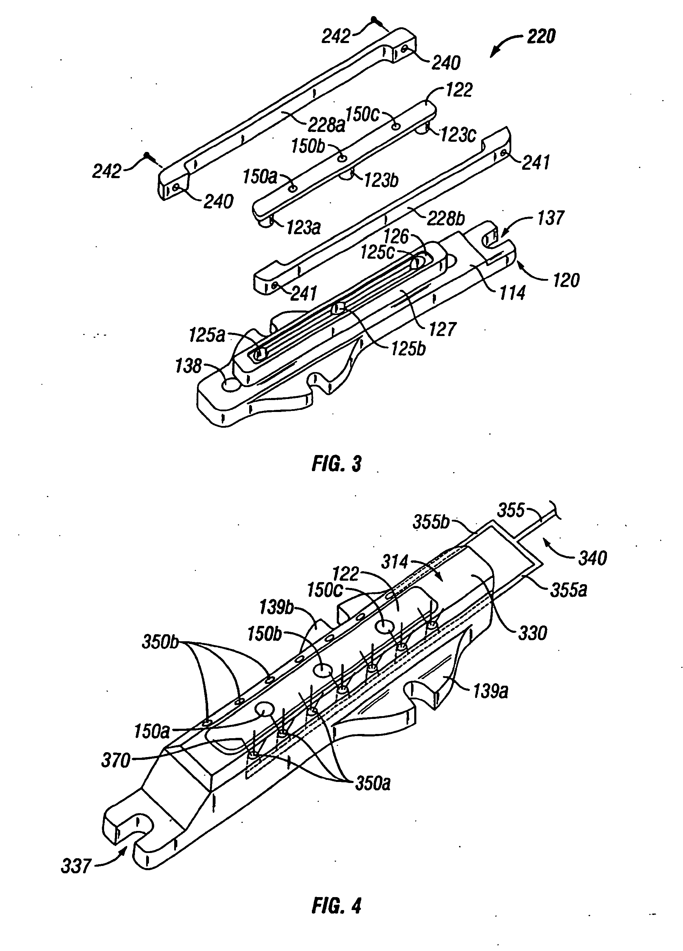

As mentioned above, the thermally conductive material 128 is also electrically non-conductive which also restricts current concentrations to between the two opposing sealing plates 122. The thermally conductive material 128 may be made from a material having a high thermal conductivity value or "k" value and minimum electrical conductively, e.g., anodized aluminum. Alternatively, the thermally conductive material 128 may also be made from or combined with a semi-resilient or elastomeric material so as not to inflict mechanical damage to the tissue during compression. Mechanical damage may also be diminished by minimizing the overall tissue contact area of the thermally conductive material 128 (See, e.g., FIG. 3). Alternatively, a spring loaded system (not shown) designed to apply pressures below critical tissue pressure limits may be employed to reduce mechanical damage of the tissue when under compression.

Other compression-reducing systems are also envisioned to avoid over-compression of tissue adjacent the sealing plates 122 and between the opposing thermally conductive materials 128, e.g., rubber-like inserts, foam or the like. Other examples of thermally conductive and electrically non-conductive materials which can be utilized to minimize thermal damage to surrounding tissue include, but are not limited to: thermally conductive plastic materials which dissipate heat along a preferred isothermal profile to the surrounding environment resulting in a lower maximum temperature and reduced formation of hot spots. Examples of such materials are commonly sold under the trademark CoolPoly^{®} by Cool Polymers, Inc., of Rhode Island and composite materials such as ALO₂.

As mentioned above, the thermally conductive material 128 includes two segments 128a and 128b which mate about the sealing plate 122 and the support step 127. More particularly, each segment 128a and 128b includes a tissue contacting surface 143a and 143b with a recessed portion 129a and 129b, respectively, along an inner peripheral edge of the tissue contacting surface 143a and 143b such that, once the two segments 128a and 128b are assembled they form a slot 141 for seating the sealing plate 122 therein. The sealing plate 122 is typically seated to lie generally flush with or below the tissue contacting surfaces 143a, 143b of the thermally conductive segments 128a and 128b. It is also envisioned that the thickness (or height relative to the insulating housing 114) of the thermally conductive material 128 proximate the recessed portions 129a, 129b is about equal to the height of the step 127 plus the thickness of the sealing plate 122 such that, once assembled, the sealing plate 122 and the thermally conductive material 128 lie substantially flush or below within the sealing plane.

The thermally conductive segments 128a and 128b may also include a series of fin-like extensions 145a, 145b, 145c and 146a, 146b, 146c, respectively, which extend laterally therefrom. It is envisioned that the fin-like extensions 145a, 145b, 145c and 146a, 146b, 146c further absorb or dissipate heat emanating from the sealing plates 122 during or after activation. The fins 145a, 145b, 145c and 146a, 146b, 146c may also be shaped and dimensioned to facilitate manufacturing and assembly, i.e., the fins 145a, 145b, 145c and 146a, 146b, 146c may be shaped to include slots 132 therein which allow passage of one or more screws 135a, 135b which attach the insulating housing 114 to the underlying electrode sealing assembly 100.

As mentioned above, the sealing plate 122 is electromechanically connected to the underlying insulating housing 114 by virtue of a series of electro-mechanical interfaces 123a, 123b and 123c which project outwardly therefrom to mate with a series of corresponding electromechanical interfaces 125a, 125b and 125c. It is envisioned that the electromechanical interfacing elements 123a, 123b, 123c and 125a, 125b, 125c maintain electrical continuity from the insulating housing 114 to the sealing plate 122. As mentioned above, once assembled and interfaced with the insulating housing 114, the thermally conductive material 128 encapsulates and further secures the sealing plate 122 atop the insulating housing 114.

A series of stop members 150a, 150b and 150c may be disposed on the tissue contacting surfaces or the inner-facing surfaces of the electrically conductive sealing plates 122 (and/or the opposite sealing plate 112 (See FIG. 1A) on jaw member 110) to facilitate gripping and manipulation of tissue and to define a gap distance between opposing jaw members 110 and 120 (or 110' and 120') during sealing. In order to achieve a desired spacing between the electrically conductive plates 112, 122 of the respective jaw members 110, 120, (i.e., gap distance) and apply the required force to properly seal tissue, at least one jaw member 110 or 120 includes at least one stop member or stop members, e.g., 150a, 150b and 150c, which limit the movement of the two opposing jaw members 110 and 120 relative to one another. The stop members, e.g., 150a, extends from the sealing plate or tissue contacting surface 122 a predetermined distance according to the specific material properties of the stop member 150a (e.g., compressive strength, thermal expansion, etc.) to yield a consistent and accurate gap distance during sealing. The gap distance between opposing sealing surfaces 112, 122 (and the sealing surface (not shown) of jaw member 110) during sealing preferably ranges from about 25 µm to about 150 µm(about 0.001 inches to about 0.006 inches) and, preferably, between about 50 µm to 75 µm (about 0.002 inches and about 0.003 inches). For larger tissue structures such as bowel, lung or intestine the gap distance ranges from about 25 µm to about 300 µm (about 0.001 inches to about 0.012 inches) and preferably from about 125 µm to about 175 µm (about 0.005 inches to about 0.007 inches).

Stop members 150a-150c are typically made from an insulative material, e.g., parylene, nylon and/or ceramic. The stop members 150a-150c can be disposed on one or both of the jaw members 110 and 120 and may be dimensioned in a variety of different shapes and sizes, e.g., longitudinal, circular, ridge-like, etc.

The non-conductive stop members 150a-150c are molded onto the sealing plates 112 and 122 (e.g., overmolding, injection molding, etc.), stamped onto the sealing plates 112 and 122, deposited (e.g., plasma deposition) onto the sealing plates 112 and 122 and/or thermally sprayed onto the surface of the sealing plates 112 and 122 (e.g., a ceramic material may be thermally sprayed) to form the stop members 150a-150c. Many different configurations for the stop members 150a-150c are discussed in detail in commonly-assigned, co-pending U.S. Application Serial No. PCT/US01/11413 entitled "VESSEL SEALER AND DIVIDER WITH NON-CONDUCTIVE STOP MEMBERS" by Dycus et al.

It is also envisioned that the thermally conductive material 128 may be dimensioned thicker than the height of step 127 and the thickness of the sealing plate 122 such that the thermally conductive material 128 acts like a stop member for maintaining a gap distance between the sealing plates 122 during activation.

In addition to keeping the pressure within a working range (i.e., about 3 kg/cm² to about 16 kg/cm²) and the gap distance within a specified range (i.e., about 25 µm (0.001 inches) to about 300 µm (0.012 inches) for large tissue structures) the electrical power should be kept within the range of about 1 W to about 350 W, about 1 Vrms to about 400 Vrms and about 0 Amps to about 5.5 Amps.

Thermal spread on each side of the sealing plates 122 is ideally kept to less than about 2mm and preferably to less than about 0.5mm to promote tissue healing. However, when sealing larger or well-vascularized tissue structures, thermal spread is acceptable to about 5mm. It is envisioned that maintaining the viability of tissue surrounding or adjacent the sealing site or fused tissue area will promote healing.

FIGS. 3 and 4 show alternate embodiments of lower jaw members 220 and 320 of the electrode sealing assembly 100 which may be utilized to reduce thermal spread to adjacent tissue during activation. More particularly, FIG. 3 shows a lower jaw member 220 which includes the same insulating housing 114 and sealing plate 122 configuration of FIGS. 2A and 2B. The thermally conductive material 228 is modified to have a reduced width which, as mentioned above, reduces the overall tissue contacting surface of the thermally conductive material 128. It is envisioned that mechanical damage may be diminished or at least maintained below critical tissue pressure limits by minimizing the overall tissue contact area of the thermally conductive material 128. Much in the same fashion as described above with respect to FIGS. 2A and 2B, the thermally conductive material 228 is secured about the sealing plate 122 and the step 127 by a series of screws 242 which mate into apertures 240 and 241 in segments 228a and 228b. As can be appreciated, the overall required width of the thermally conductive material 228 may be dependent upon type of tissue being sealed or the thickness of the tissue being sealed. Step 127 may include a reliefed portion 126 disposed therein which seats or aligns the sealing plate 122 during assembly.

FIG. 4 shows yet another possible configuration of the lower jaw member 320 of the electrode sealing assembly 100 (or 100') designed to reduce thermal spread to adjacent tissue. In this embodiment, a thermally conductive material is not utilized as the heat absorbing material or heat sink, but, rather, an active cooling system 340 surrounds the sealing plate 122 to reduce heat dissipation to surrounding tissue. More particularly, insulating housing 314 includes a series of ducts or tubes 355, 355a and 355b disposed therethrough. The coolant ducts 355a, 355b are configured to transport a coolant 370 to the insulating housing 314 to dissipate heat away from surrounding tissue adjacent the sealing plates 122 to actively cool the tissue during activation which reduces thermal spread.

The coolant ducts 355, 355a, 355b supply active cooling liquid (preferably, non-electrically conductive cooling liquid) or gas (e.g., air) 370 through at least one of a series of nozzles or ports 350a and 350b disposed on an upper surface 330 of the insulating housing 314. The nozzles or ports 350a and 350b are located immediately adjacent the sealing plate 122 and extend longitudinally on opposite sides thereof, i.e., ports 350a extend along one side of the sealing plate 122 and ports 350b extend along the opposite side of the sealing plate 122. The nozzles or ports 350a and 350b are configured to discharge the coolant 370 to an environment proximate the electrode sealing assembly 100 (or 100').

As can be appreciated, the sealing system 340 supplies coolant (liquid or gas (e.g., air)) 370 to the tissue areas adjacent the sealing plates 122 to actively cool the tissue during activation which reduces thermal spread. With respect to this particular embodiment and compared to the embodiments of FIGS. 2A-3, the insulating housing 314 encapsulates the sealing plate 122 by virtue of a mechanical connection or manufacturing process, e.g. stamp molding or injection molding.

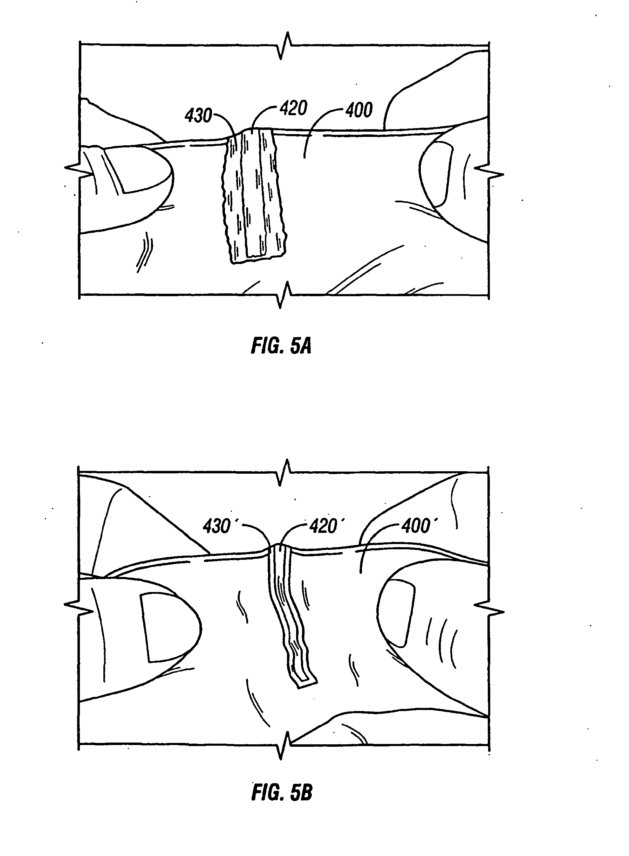

FIGS. 5A and 5B show a side-by-side comparison of the resulting tissue seals 420 and 420' utilizing a prior vessel sealing instrument (See FIG. 5A) and a vessel sealing instrument designed to reduce thermal spread to adjacent tissue 400 according to the present disclosure (See FIG. 5B). More particularly and with respect to FIG. 5A, there is some notable thermal damage 430 to adjacent tissue 400 proximate the tissue seal 420. FIG. 5B shows the resulting seal 420' utilizing one of the various electrode assemblies 100 (or 100') described herein. A more uniform and narrower seal 420' is evident with a significant reduction of thermal damage 430' to adjacent tissue 400. It is envisioned that reducing thermal damage to adjacent tissue 400 can improve healing especially in sensitive tissue areas, e.g., small and large intestines. As mentioned above, the thermal spread is preferably kept to about 2mm with sensitive large tissues and vessels and about 5mm with non-sensitive tissues and vessels.

FIG. 6 shows an alternative electrode sealing assembly 500 which is also designed to reduce thermal spread to adjacent tissue. More particularly, electrode sealing assembly 500 includes upper and lower jaws 510 and 520, respectively, which each include a thermally conductive, electrically insulative material 530a and 530b, e.g., a so-called "cool polymer" material, disposed on (or within) the respective tissue sealing plates, 512 and 522. The cool polymers 530a, 530b may be centrally disposed within each sealing plate 512 and 522, respectively. It is envisioned that the cool polymers 530a and 530b will act as heat sinks (i.e., absorb heat) during activation which will limit the thermal spread to adjacent tissue 400. Examples of cool polymers include thermally conductive plastic materials which dissipate heat in a more isothermal profile to the surrounding environment resulting in a lower maximum temperature and reduced formation of hot spots such as materials commonly sold under the trademark CoolPoly^{®} by Cool Polymers, Inc., of Rhode Island. Alternatively, certain known ceramic materials may also be used to reduce tissue effects.

FIG. 7 shows yet another electrode sealing assembly 600 which is also designed to reduce thermal spread to adjacent tissue 400. More particularly, electrode sealing assembly 600 includes upper and lower jaw members 610 and 620, respectively which are designed to engage tissue 400 therebetween. Each of the jaw members 610 and 620 includes a recessed portion 630 and 640, respectively which is dimensioned to allow bulging portions 450a and 450b of the tissue 400 to bulge into each respective jaw member 610 and 620 when the tissue 400 is under compression. It is envisioned that the moisture in the less-compressed tissue bulges 450a and 450b essentially acts as a heat sink to absorb heat during activation and reduce thermal spread to surrounding tissue.

It is envisioned that the jaw members 110 and 120 may be curved in order to reach specific anatomical structures and promote more consistent seals for certain procedures. For example, it is contemplated that dimensioning the jaw members 110 and 120 at an angle of about 45 degrees to about 70 degrees is preferred for accessing and sealing specific anatomical structures relevant to prostatectomies and cystectomies, e.g., the dorsal vein complex and the lateral pedicles. Other angles may be preferred for different surgical procedures.

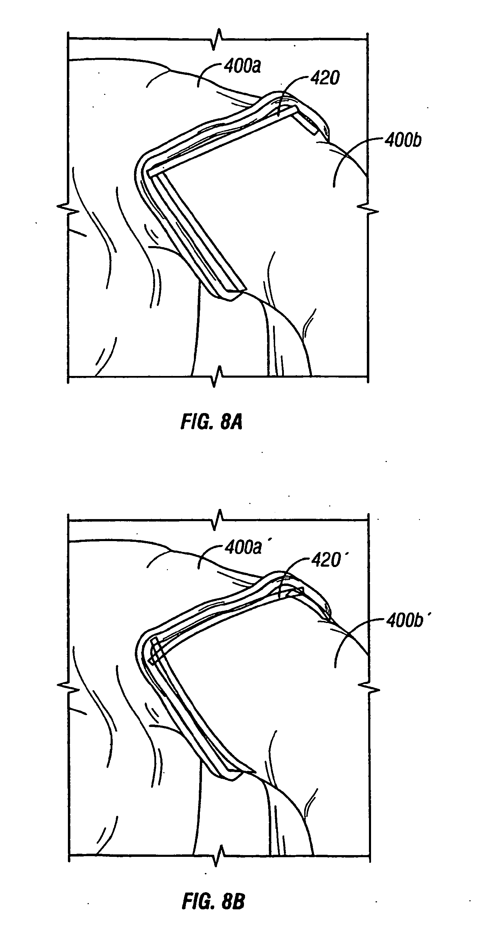

For example and as best shown in FIGS. 8A and 8B, it may be preferable to use a curved jaw member (not shown) for an end-to-end anastomosis of bowel tissues. FIG. 8A shows the resulting seal 420 of an end-to-end anastomosis of two bowel segments 400a and 400b utilizing a straight pair of jaw members. FIG. 8B shows a resulting seal 420' of an end-to-end anastomosis of two bowel segments 400a' and 400b' utilizing a curved pair of jaw members. As can be appreciated the resulting seal 420' from the curved pair of jaw members tends to more closely conform to the general contours of the two tissue segments 400a' and 400b' which is envisioned will promote tissue healing around the anastomosis site.

It is also envisioned that the jaw members 110 and 120 may be tapered which is advantageous for two reasons: 1) the taper will apply constant pressure for a constant tissue thickness at parallel; 2) the thicker proximal portion of each jaw member 110 and 120 will resist bending due to the reaction force of the tissue 400.

It is also envisioned that the above forceps 10 (or 10') may be utilized in connection with a closed-loop RF control system which optimizes sealing based upon pre-surgical conditions or changes in physical or electrical conditions during sealing. One example of a closed-loop control system is described in commonly-owned U.S, Patent Application Serial No. 10/427,832 filed on May 1, 2003 entitled "METHOD AND SYSTEM FOR CONTROLLING OUTPUT OF RF MEDICAL GENERATOR" and commonly-owned U.S. Patent Application Serial No. 10/835,657 filed on April 30, 2004 entitled "METHOD AND SYSTEM FOR PROGRAMMING AND CONTROLLING AN ELECTROSURGICAL GENERATOR SYSTEM". In general, the closed-loop control, system includes a user interface for allowing a user to select at least one pre-surgical parameter, such as the type of surgical instrument operatively connected to the generator, the type of tissue and/or a desired surgical effect. A sensor module is also included for continually sensing at least one of electrical and physical properties proximate the surgical site and generating at least one signal relating thereto.

The closed loop control system also includes a control module for continually receiving or monitoring surgical parameters and each of the signals from the sensor module and processing each of the signals in accordance with a desired surgical effect using a microprocessor, computer algorithm and/or a look-up table. The control module generates at least one corresponding control signal relating to each signal from the sensor module(s), and relays the control signal to the electrosurgical generator for controlling the generator. The closed loop system may be employed in a feedback circuit or part of a surgical method for optimizing a surgical seal. The method includes the steps of: applying a series of electrical pulses to the surgical site; continually sensing electrical and physical properties proximate the surgical site; and varying pulse parameters of the individual pulses of the series of pulses in accordance with the continually-sensed properties. Alternatively, the signal may be continuous.

It is also contemplated that the sealing surfaces 122 of the jaw members 110 and 120 can be made from or coated with non-stick materials to reduce tissue adhesion. Alternatively, the jaw members 110 and 120 may be surface treated, roughened, to reduce sticking, e.g., bead blasting, stamping. When utilized on the sealing surfaces 122, these materials provide an optimal surface energy for eliminating sticking due in part to surface texture and susceptibility to surface breakdown due to electrical effects and corrosion in the presence of biologic tissues. It is envisioned that these materials exhibit superior non-stick qualities over stainless steel and should be utilized on the forceps 10 (or 10') in areas where the exposure to pressure and RF energy can create localized "hot spots" more susceptible to tissue adhesion. As can be appreciated, reducing the amount that the tissue "sticks" during sealing improves the overall efficacy of the instrument. Controlling tissue cooling may also reduce adhesion or buildup of tissue on the electrodes and also assist during the formation of the tissue seal, e.g., cross-linking or other chemical bonding, during the reformation or renaturation of collagen.

The non-stick materials may be manufactured from one (or a combination of one or more) of the following "non-stick" materials: nickel-chrome, chromium nitride, MedCoat 2000, Inconel 600, tin-nickel or various nitride coatings which include, but are not limited to, TiN, ZrN, TiAlN and CrN. For example, high nickel chrome alloys, Ni200, Ni201 (~100% Ni) may be made into electrodes or sealing surfaces by metal injection molding, stamping, machining or any like process. Also and as mentioned above, the sealing surfaces 122 may also be "coated" with one or more of the above materials to achieve the same result, i.e., a "non-stick surface".

It is further envisioned that thermal spread may be reduced by altering the physical dimensions of the insulating housing 114. For example, in some cases it may be preferable to manufacture the insulating housing 114 from a variety of materials (either alone or in combination) which include: nylons and syndiotactic polystryrenes such as QUESTRA^{®} manufactured by DOW Chemical; Polybutylene Terephthalate (PBT); Polycarbonate (PC); Acrylonitrile Butadiene Styrene (ABS); Polyphthalamide (PPA); Polymide, Polyethylene Terephthalate (PET); Polyamide-imide (PAI); Acrylic (PMMA); Polystyrene (PS and HIPS); Polyether Sulfone (PES); Aliphatic Polyketone; Acetal (POM) Copolymer; Polyurethane (PU and TPU); Nylon with Polyphenylene-oxide dispersion; and Acrylonitrile Styrene Acrylate.

It is also contemplated that only one of the two jaw members 110 and 120 may include one of the aforedescribed mechanisms or configurations for reducing thermal spread. For example and with reference to FIGS. 2A, 2B and 3, it is contemplated that only the lower jaw member 120, 220 may include the thermally conductive material 128, 228 disposed between the insulating housing 114 and the sealing plate 122. With reference to FIG. 4, only the lower jaw member 320 may include the active cooling system 340. With reference to FIG. 6, only the top jaw member 510 may be configured to house a cool polymer 530a for reducing thermal spread to adjacent tissue 400. Likewise and with reference to FIG. 7, only the upper jaw member 610 may include a recessed area 630 for receiving bulging tissue 450a. It is further contemplated that the above configurations may be used in combination to reduce thermal spread to adjacent tissue. For example, a cool polymer 530a may be used in combination with the thermally conductive material 128 of FIG. 2A or used in replace of the thermally conductive material 128 of FIG. 2A depending upon a particular purpose.

It is envisioned that the forceps 10 or 10' may be designed such that it is fully or partially disposable depending upon a particular purpose or to achieve a particular result. For example, electrode sealing assembly 100 may be selectively and releasably engageable with the distal end 14 of the shaft 12 and/or the proximal end 16 of shaft 12 may be selectively and releasably engageable with the housing 20 and the handle assembly 30. In either of these two instances, the forceps 10 would be considered "partially disposable" or "reposable", i.e., a new or different electrode sealing assembly 100 (or electrode sealing assembly 100 and shaft 12) selectively replaces the old jaw assembly 110 as needed.

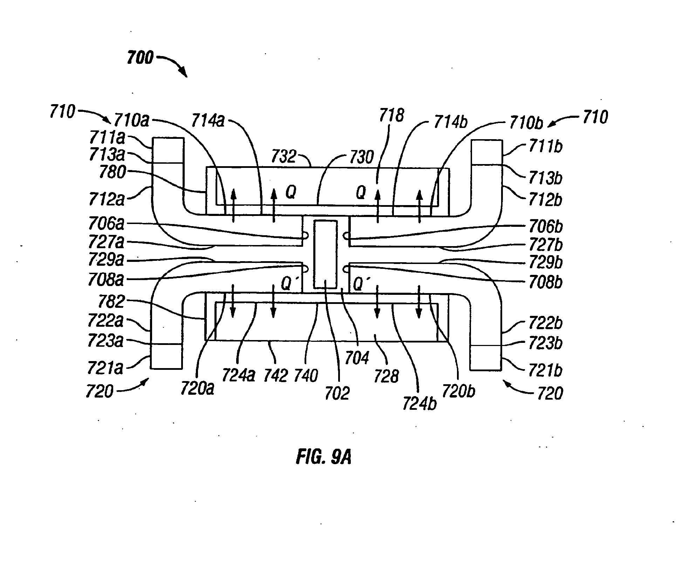

Another embodiment of an electrode cooling system for an electrode assembly 700 according to the present disclosure is illustrated in FIG. 9A. More particularly, FIG. 9A shows an end view of a distal end of lower electrode jaw member 720 and a distal end of upper electrode jaw member 710 of electrode assembly 700 adapted for use as a bipolar forceps 10. The upper electrode jaw member 710 includes upper electrically insulating portions 711 a, 711b joined at edges 713a, 713b to contact electrically conductive seal plates 712a, 712b. The lower electrode jaw member 720 includes lower electrically insulating portions 721 a, 721 b joined at edges 723a, 723b to contact electrically conductive seal plates 722a, 722b. A knife blade 702 is shown disposed within a knife slot 704 formed by inward lateral side edges 706a and 706b of the electrically conductive seal plates 712a and 712b and by inward lateral side edges 708a and 708b of the electrically conductive seal plates 722a and 722b. The jaw members 710 and 720 have a generally U-shaped cross-section with a generally flat central portion 710a, 710b, 720a, 720b, in the electrically conductive seal plates 712a, 712b, and 722a, 722b, respectively.

During the tissue sealing process, heat Q is generated on inner surface 727a, 727b in the generally flat central portion 710a, 710b of electrically conductive seal plates 712a and 712b. Similarly, heat Q' is generated on inner surface 729a, 729b in the generally flat central portion 720a, 720b of electrically conductive seal plates 722a and 722b..

At least one of the jaw members 710 and 720 includes a thermoelectric plate such that heat generated by at least one of the jaw members is transferred away from the tissue via the thermoelectric plate. More particularly, a first surface 730 of an upper thermoelectric (TEC) plate 718 and an outer surface 714a, 714b of the upper electrically conductive seal plates 712a, 712b in the generally flat central portion 710a, 710b have a thermally conductive, electrically insulating material 780 disposed therebetween. Correspondingly, a first surface 740 of a lower thermoelectric (TEC) plate 728 and an outer surface 724a, 724b of the lower electrically conductive seal plates 722a, 722b in the generally flat central portion 720a, 720b have a thermally conductive, electrically insulating material 782 disposed therebetween.

The heat Q generated on inner surface 727a, 727b of upper jaw member 710 is transferred through the upper electrically conductive seal plates 712a, 712b and through the thermally conductive, electrically insulating material 780 to the first surface 730 of the upper TEC plate 718 where the heat Q is transferred to the TEC plate 718.

Similarly, the heat Q generated on inner surface 729a, 729b of upper jaw member 720 is transferred through the lower electrically conductive seal plates 722a, 722b and through the thermally conductive, electrically insulating material 782 to the first surface 740 of the lower TEC plate 728 where the heat Q is transferred to the TEC plate 728.

It is contemplated that in most cases of electrosurgery, both of the jaw members 710 and 720 would include their respective TEC plates 718 and 728 for cooling purposes. Furthermore, those skilled in the art will recognize that TEC plates 718 and 728 may be alternatively referred to as solid state heat pumps or Peltier coolers.

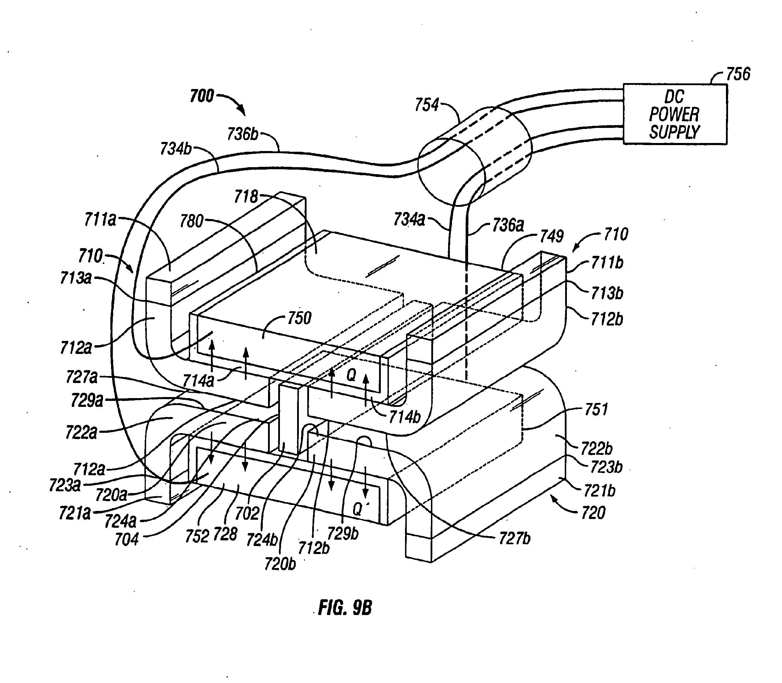

As shown in FIG. 9B, electrical lead 734a is connected to a proximal end 749 of upper TEC plate 718, while electrical lead 734b is connected to a distal end 750 of upper TEC plate 718. Similarly, electrical lead 736a is connected to a proximal end 751 of lower TEC plate 728, while electrical lead 736b is connected to a distal end 752 of lower TEC plate 728. The leads 734a, 734b, 736a, 736b are routed through a conduit or cable 754 to a direct current (DC) power supply 756. As noted previously, during the tissue sealing process, heat Q is generated on inner surface 727a, 727b in the generally flat central portion 710a, 710b of upper seal plates 712a, 712b. Similarly, heat Q' is generated on inner surface 729a, 729b in the generally flat central portion 720a, 720b of lower seal plate 722a, 722b.

The TEC plates 718 and 728 provide the capability of directing this heat Q away from the inner surfaces 727a, 727b and 729a, 729b depending upon direction of current flow through the electrical leads. In most cases of electrosurgery, the TEC plates would be used for cooling rather than heating. To achieve cooling, direction of current is controlled by the power supply 756 and current is directed through the TEC plates 718 and 728 such that the heat Q from the seal plates 712a, 712b, 722a, 722b is directed away from the tissue and towards the opposite end of the TEC plates 718 and 728. As can be appreciated, the heat Q generated during tissue sealing by the electrodes 710 and 720 is transferred away from the tissue and is not transmitted to surrounding tissue, thus reducing collateral *damage* to tissue. The thermally conductive, electrically insulating materials 780, 782 may be made of a cool polymer as described previously which prevents electrical continuity between the DC power supply 756 and an AC power supply from the previously discussed source of electrosurgical energy e.g., an electrosurgical generator (not shown) via plug 300 and electrical cable 310 (see FIGS. 1A and 1B).

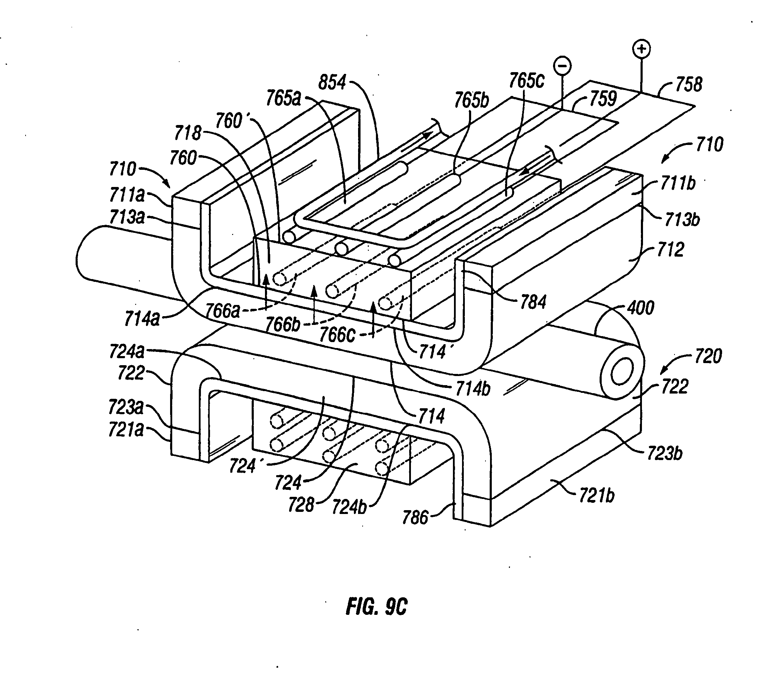

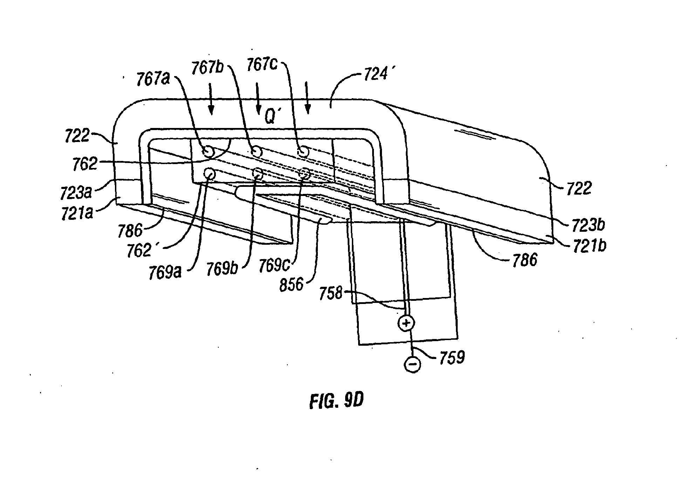

FIGS. 9C and 9D show one particularly useful embodiment according to the present disclosure wherein TEC plate 718 is utilized to dissipate heat from the jaw members 710 and 720 during tissue treatment. More particularly, and with specific reference to jaw member 710, the jaw member 710 includes upper electrically insulating portions 711 a and 711 b joined at edges 713a, 713b to contact an electrically conductive seal plate 712. TEC plate 718 is disposed within jaw member 710 on the opposite side 714' of tissue engaging surface 714 of the electrically conductive sealing plate 712. A thermally conductive electrically insulating material 784 is disposed between the TEC sealing plate 718 and sealing plate 712 on outer surfaces 714a and 714b of the sealing plate 712. The plate 718 includes first and second sides 760 and 760', respectively. Side 760 abuts the opposite end 714' of sealing plate 712. A series of electrical leads 765a, 765b, and 765c are connected to the second side 760' while a series of electrical leads 766a, 766b, and 766c are connected to the first side 760.

It is envisioned that a first electrical potential 758 may be selectively transmitted through leads 765a, 765b and 765c and a second electrical potential 759 may be selectively transmitted through leads 766a, 766b, and 766c such that different electrical potentials are created on opposite sides of the plate 718. As can be appreciated, heat Q in this instance may be directed proximally for absorption by a second heat sink, e.g., cool polymer, a fluid through one or more ducts 854 disposed in contact with TEC plate 718, or another TEC plate.

Jaw member 720 is configured in much the same manner and includes similar elements for directing heat Q proximately. More particularly, and with specific reference to jaw member 720, the jaw member 720 includes lower electrically insulating portions 721 a and 721 b joined at edges 723a, 723b to contact an electrically conductive seal plate 722. TEC plate 728 is disposed within jaw member 720 on the opposite side 724' of tissue engaging surface 724 of the electrically conductive sealing plate 722. A thermally conductive, electrically insulating material 786 is disposed between the sealing plate 722 and the TEC plate 728 on outer surfaces 724a and 724b of the sealing plate 722. The plate 728 includes first and second sides 762 and 762', respectively. Side 762 abuts the opposite end 724' of sealing plate 722. A series of electrical leads 767a, 767b, and 767c are connected to the first side 762 while a series of electrical leads 769a, 769b and 769c are connected to the second side 762'.

The thermally conductive, electrically insulating materials 784, 786 may be made of a cool polymer as described previously which prevents electrical continuity between the DC power supply 756 and an AC power supply from the previously discussed source of electrosurgical energy.

It is envisioned that first electrical potential 758 may be selectively transmitted through leads 767a, 767b and 767c and second electrical potential 759 may be selectively transmitted through leads 769a, 769b, and 796c such that different electrical potentials are created on opposite sides of the plate 728. As can be appreciated, heat Q' in this instance may be directed proximally for absorption by a second heat sink, e.g., cool polymer, a fluid through one or more ducts 856 disposed in contact with TEC plate 728, or another TEC plate. As can be appreciated, the two jaw members 710, 720 cooperate to remove excess heat from the tissue to reduce collateral tissue effects during sealing.

FIG. 10A shows a proximal end of the electrode assembly 700 configured in one particularly useful embodiment for forced convection cooling of the upper electrode jaw members 710 and lower electrode jaw members 120. FIG. 10A is in all respects identical to FIG. 9A except that electrode assembly 700 is configured for forced convection cooling of the upper seal plates 712a, 712b and lower seal plates 722a, 722b. More particularly, a heat sink 818 is disposed in direct contact with a second surface 732 of thermoelectric cooling plate 718. A coolant or cooling line 850 is disposed through or embedded within heat sink 818. The coolant line 850 has a coolant supply end 850a and a coolant return end 850b projecting from a proximal end of the heat sink 818.

Similarly, a heat sink 828 is disposed in direct contact with a second surface 742 of thermoelectric cooling plate 728. A coolant or cooling line 852 is disposed through or embedded within heat sink 828. The coolant line 852 has a coolant supply end 852a and a coolant return end 852b projecting from a proximal end of the heat sink 828.

FIG. 10B shows a front perspective view of the electrode assembly 700 of FIG. 10A as configured for forced convection cooling of the upper seal plates 712a, 712b and lower seal plates 722a, 722b. More particularly, the heat sink 818 is disposed in direct contact with the second surface 732 of thermoelectric cooling plate 718. The coolant line 850 is disposed through or embedded within heat sink 818. The coolant line 850 has coolant supply end 850a and coolant return end 850b projecting from a proximal end 838 of the heat sink 818. The coolant line 850 may form a U-bend 850c proximate to a distal end 842 of heat sink 818.

Similarly, heat sink 828 is disposed in direct contact with the second surface 742 of thermoelectric cooling plate 728. The coolant line 852 is disposed through or embedded within heat sink 828. The coolant line 852 has a coolant supply end (not shown) and a coolant return end (not shown) projecting from a proximal end 840 of the heat sink 828. The coolant line 852 may form a U-bend 852c proximate to a distal end 844 of heat sink 828 in an analogous manner as shown with respect to U-bend 850c of coolant line 850 in heat sink 818.

In the foregoing embodiment, it is particularly suitable for the coolant lines 850 and 852 to contain an active cooling fluid (e.g., a thermally conductive, non-electrically conductive cooling liquid or a gas, e.g., air). In particular, the cooling fluid may include a liquid coolant such as water or a non-conductive fluid such as a medicinal or biocompatible fluid. However, a gas such as, but not limited to, air, nitrogen or carbon dioxide (preferably at ambient or above ambient pressure conditions) may be applied under forced flow conditions. Alternatively, coolant lines 850 and 852 may also be filled with a stagnant substance such as a below ambient temperature gas (including air, nitrogen or carbon dioxide), or a liquid or solid or frozen substance such as water ice or dry ice (solid carbon dioxide).

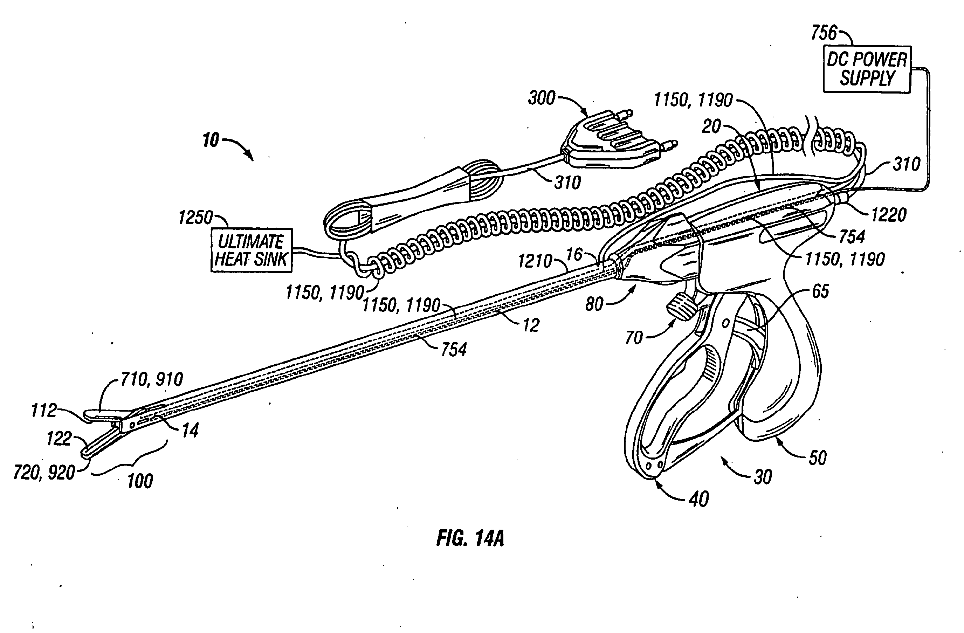

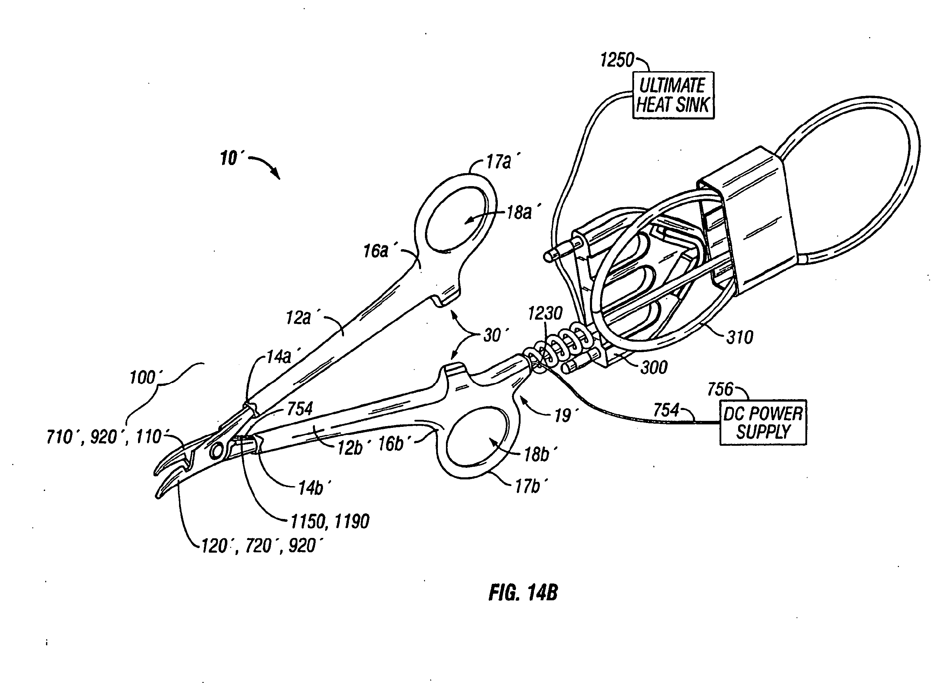

Coolant applied to coolant supply lines 850 and 852 removes the heat Q generated during the tissue sealing process. As discussed in more detail below with respect to FIGS. 14A and 14B, the heat sinks 818 and 828 may be configured to be coupled to an ultimate heat sink for transferring heat from the jaw members 710 and 720. More particularly, via the coolant supply ends 850a, 852a, the coolant or cooling lines 850 and 852 may be configured to receive the coolant to transfer the heat from the respective thermoelectric cooling plates 718 and 728. Furthermore, via the coolant return ends 850b, 852b, the coolant or cooling lines 850 and 852 may be configured to be coupled to an ultimate heat sink via the forceps 10.

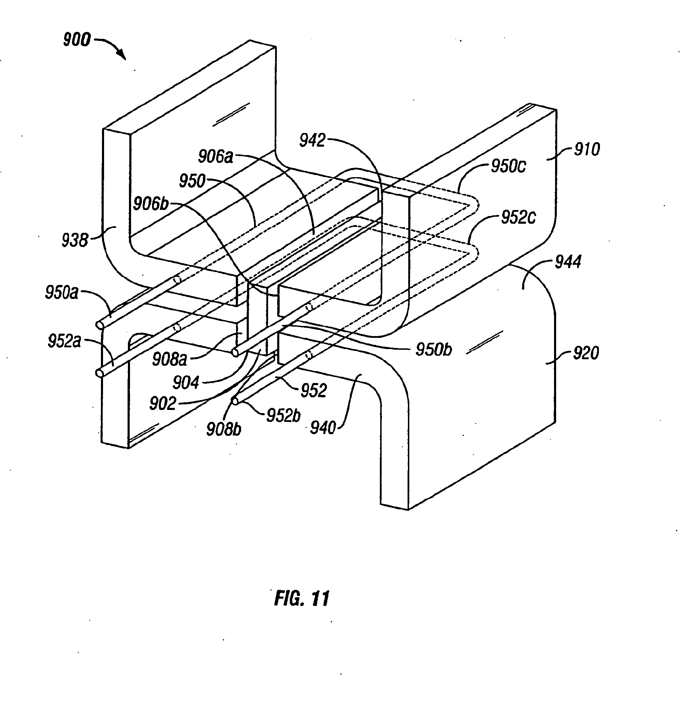

FIG. 11 shows yet another embodiment of an electrode cooling system for an electrode assembly 900 according to the present disclosure. More particularly, FIG. 11 shows a proximal end 938 of an upper electrode jaw member 910 and a proximal end 940 of a lower electrode jaw member 920 of electrode assembly 900 adapted to bipolar forceps 10. A knife blade 902 is shown disposed within a knife slot 904 formed by the inward lateral side edges 906a and 906b of the upper jaw member 910 and by the inward lateral side edges 908a and 908b of the lower jaw member 920. The jaw members 910 and 920 have a generally U-shaped cross-section.

At least one of the jaw members 910 and 920 includes a cooling line disposed therethrough or embedded therein. More particularly, a coolant or cooling line 950 may be disposed or embedded within upper electrode jaw member 910. The coolant line 950 has a coolant supply end 950a and a coolant return end 950b projecting from a proximal end 938 of the upper jaw member 910. The coolant line 950 may form a U-bend 850c proximate to a distal end 942 of upper jaw member 910.

Similarly, a coolant or cooling line 952 may be disposed or embedded within lower electrode jaw member 920. The coolant line 952 has a coolant supply end 952a and a coolant return end 952b projecting from a proximal end 940 of the lower jaw member 920. The coolant line 952 may form a U-bend 952c proximate to a distal end 944 of lower jaw member 920.

The coolant lines 950 and 952 may be configured to receive a coolant to transfer heat from jaw members 910 and/or 920. In a similar manner to the previous embodiment described above, it is particularly suitable for the coolant received by the coolant lines 950 and 952 to be an active cooling fluid (preferably, a non-electrically conductive cooling liquid or a gas, e.g., air).

Coolant applied to coolant supply lines 950 and 952 removes the heat Q generated during the tissue sealing process. As discussed in more detail below with respect to FIGS. 14A and 14B, the coolant supply ends 950a, 952a and coolant return ends 950b, 952b may be coupled to an ultimate heat sink via the forceps 10.

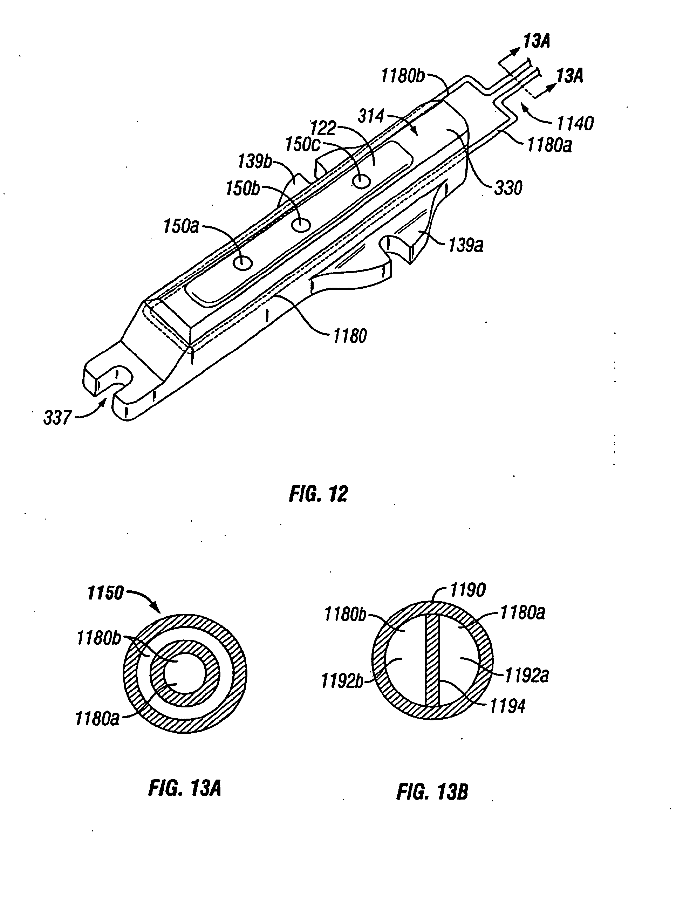

FIG. 12 is an enlarged, perspective view of still another embodiment of the electrode sealing assembly of FIG. 4. More particularly, FIG. 12 shows yet another possible configuration of the lower jaw member 320 of the electrode sealing assembly 100 (or 100') designed to reduce thermal spread to adjacent tissue. This embodiment is in all respects identical to the embodiment disclosed by FIG. 4 except that open active cooling system 340 with a common supply line 355, which branches out into coolant lines 355a and 355b to supply coolant 370 through the series of nozzles or ports 350a and 350b located on an upper surface 330 of the insulating housing 314, is replaced by closed active coolant system 1140 which includes a U-shaped continuous coolant loop 1180 having a coolant supply end 1180a and a coolant return end 1180b. The coolant supply loop 1180 is disposed through or embedded within the insulating housing 314 surrounding the sealing plate 122. The coolant loop 1180 is configured to receive the coolant 370, which is, typically, a non-electrically conductive cooling liquid or gas (e.g., air) such as previously described. The active coolant 370 is caused to flow through the coolant loop 1180 to reduce heat dissipation to surrounding tissue which is generated by the tissue sealing process in sealing plate 122. As is the case of the embodiment of FIG. 4, a thermally conductive material is not utilized as the heat absorbing material or heat sink, but, rather, the active cooling system 1140 surrounds the sealing plate 122. As is discussed in more detail later with respect to FIGS. 14A and 14B, the coolant loop 1180 transports the coolant to an ultimate heat sink for dissipating heat away from surrounding tissue.

With respect to this particular embodiment and compared to the embodiments of FIGS. 2A, 2B, 3 and 4, again, the insulating housing 314 encapsulates the sealing plate 122 by virtue of a mechanical connection or manufacturing process, e.g. stamp molding or injection molding.

FIG. 13A is a cross-sectional end view of one embodiment of cooling loop 1180 for the electrode cooling assemblies of FIG. 12. More particularly, the ends 1180a and 1180b of the cooling loop 1180 are joined together in a common cooling line 1150. The common cooling line 1150 includes typically an inner tubular shaped conduit which can function as either supply line 1180a or return line 1180b, and an outer concentrically arranged tubular shaped conduit which can function conversely as either return line 1180b or supply line 1180a, respectively.

FIG. 13B is a cross-sectional end view of an alternate embodiment of a cooling line for the electrode assemblies of FIG. 12. More particularly, in a similar manner to the embodiment of FIG. 13A, the ends 1180a and 1180b of the cooling loop 1180 are again joined in a common cooling line designated as 1190. However, the common cooling line 1190 includes a generally tubular configuration which is segmented into two inner flow channels 1192a and 1192b via a partition 1194. The inner flow channel 1192a can function as either supply line 1180a or return line 1180b, while conversely, the inner flow channel 1192b can function as either return line 1180b or supply line 1180a, respectively.

Those skilled in the art will recognize that the coolant loops 850 and 852, and 950 and 952 (see FIGS. 10A, 10B and 11) may be configured in an analogous manner as common cooling lines 1150 and 1190.

FIG. 14A is a perspective view of the endoscopic bipolar forceps of FIG. 1A which is configured to support the common cooling lines 1150 and 1190 (see FIG. 12, FIG. 13A and FIG. 13B). More particularly, the forceps 10 includes the shaft 12 which has a distal end 14 dimensioned to mechanically engage the end effector assembly 100 and a proximal end 16 which mechanically engages the housing 20 proximate the rotating assembly 80. The cooling line 1150, or 1190 extends from the upper and lower jaws, e.g., jaw members 710, 720, 910, 920 through the shaft 12 and through the housing 20 at a port 1210 proximate the shaft 12 from which the cooling line 1150, or 1190 emerges at a port 1220 in the housing 20 proximate the electrosurgical cable 310. Alternatively, the cooling line 1150, or 1190, may be configured to bypass the housing 20 and only emerges from the shaft 12 at port 1210. Typically, in either embodiment, the cooling line 1150 or 1190 is coiled around the electrosurgical cable 310 to a convenient point at which it is directed to an ultimate heat sink 1250. The cable 754 which provides DC power to the TEC plates 718 and 728 as previously described extends from the TEC plates 718 and 728 through the shaft 12 and through the housing 20 from which cable 754 emerges at port 1220 (or a separate port) to connect to the DC power supply 756. It is contemplated that the forceps 10 described with respect to FIGS. 14A and as follows in FIG. 14B may be utilized with any of the aforementioned end effector assemblies and jaw members described herein.

More particularly, FIG. 14B is a perspective view of the open bipolar forceps of FIG. 1B which is configured to support the cooling line of FIG. 10, FIG. 11B and FIG. 11C. As disclosed previously with respect to FIG. 1B, open forceps 10' includes a pair of elongated shaft portions 12a', 12b' each having a proximal end 16a' and 16b', respectively, and a distal end 14a' and 14b', respectively. The forceps 10' includes jaw assembly 100' which attaches to the distal ends 14a' and 14b' of shafts 12a' and 12b', respectively. Jaw assembly 100' includes an upper jaw member 710' or 910' and a lower jaw member 720' or 920' which are movable relative to one another to grasp tissue therebetween. Those skilled in the art will recognize that upper jaw members 710' and 910' are substantially identical to upper jaw member 710 and 910, respectively, except for being configured to adapt to the open forceps 10'. Similarly, those skilled in the art will recognize that lower jaw members 720' and 920' are substantially identical to upper jaw member 720 and 920, respectively, except for being configured to adapt to the open forceps 10'.

Each shaft 12a' and 12b' includes a handle 17a' and 17b' disposed at the proximal end 16a' and 16b' thereof which each define a finger hole 18a' and 18b', respectively, therethrough for receiving a finger of the user. As can be appreciated, finger holes 18a' and 18b' facilitate movement of the shafts 12a' and 12b' relative to one another which, in turn, pivot the jaw members 110' and 120' from the open position wherein the jaw members 110' and 120' are disposed in spaced relation relative to one another for manipulating tissue to a clamping or closed position wherein the jaw members 110' and 120' cooperate to grasp tissue therebetween.

One of the shafts, e.g., 12b', includes a proximal shaft connector /flange 19' which is designed to connect the forceps 10' to a source of RF energy (not shown) via an electrosurgical cable 310 and plug 300. Although the details relating to the inner-working electrical connections and various components of forceps 10' are disclosed in commonly-owned U.S. Patent Application No. 10/369,894, it is disclosed herein that cooling line 1150 or 1190 and electrical cable 754 extends from the upper and lower jaw members 110' and 120' through the shaft 12b' to the proximal shaft/connector flange 19' which interfaces with electrosurgical cable 310. The cooling line 1150 or 1190 emerges from the flange 19' at a port 1230 proximate the power cord 310. Typically, the cooling line 1150 or 1190 is coiled around the electrosurgical cable 310 to a convenient point at which it is directed to the ultimate heat sink 1250. The electrical cable 754 emerges at the port 1230 from which it extends to connect to DC power supply 756.

Although it is preferable that jaw members 110 and 120 meet in parallel opposition, and, therefore, meet on the same plane, in some cases it may be preferable to slightly bias the jaw members 110 and 120 to meet each other at the distal end such that additional closure force on the handles is required to deflect the electrodes in the same plane. It is envisioned that this could improve seal quality and/or consistency. Alternatively, the jaws members 110 and 120 may be configured to close in a heel-based manner or in an independently floating (with respect to parallel) fashion.

It is envisioned that while the jaw members 710, 710', 910, 910' and 720, 720', 920, 920' are configured for dissipating heat generated by electrosurgical RF power, the cooling members disclosed herein (i.e., thermoelectric plates 718 and 728, corresponding heat sinks 818 and 828 and the cooling lines 850, 852, 950, 952; and the cooling loops 340, 1150 and 1190 for cooling the insulating housing 314) may be adapted as well to other heating modalities. Such other heating modalities include, but are not limited to, ultrasonic, capacitive or thermoelectric heating power sources.

## Claims

1. An electrode sealing assembly (700) designed for use with an electrosurgical instrument for sealing tissue, comprising:
first and second jaw members (710, 720) being movable from a first position in spaced relation relative to one another to at least one second position for grasping tissue therebetween, the jaw members including:
electrically conductive sealing plates (712a, 712b) disposed in opposing relation to one another, the electrically conductive sealing plates having respective tissue contacting surfaces (727a, 727b) for grasping and sealing tissue therebetween when the jaw members are in the second position, at least one jaw member including:
a thermoelectric cooling plate (718, 728), said thermoelectric cooling plate including first and second electrical connections (734a, 734 b, 736a, 736b) disposed on opposite sides of the thermoelectric cooling plate, said first connection being configured to selectively transmit a first electrical potential and said second connection being configured to selectively transmit a second electrical potential such that heat generated by the sealing plates is transferred away from the tissue via the thermoelectric cooling plate;
a first surface (730, 740) of the thermoelectric cooling plate and an outer surface of the sealing plate having a thermally conductive, electrically insulating material (780, 782) disposed therebetween so that the heat generated on the tissue-contacting surface of the electrically conductive sealing plate is transferred through the sealing plate and through the thermally conductive, electrically insulating material to the first surface of the thermoelectric cooling plate; **characterized in that** the at least one jaw member further includes a heat sink (818, 828) disposed in direct contact with a second surface (732, 742) of the thermoelectric cooling plate, wherein the heat sink includes a coolant line (850, 852) disposed through the heat sink (818, 823).

2. An electrode sealing assembly according to claim 1, wherein the heat sink is configured to be coupled to a second heat sink (1250) for transferring heat from the at least one of the jaw members.

3. An electrode sealing assembly according to claim 1 or 2, wherein the coolant line is configured to receive a coolant to transfer heat from the thermoelectric cooling plate.

4. An electrode sealing assembly according to claim 3, wherein the coolant is a thermally conductive, non-electrically conductive fluid.

5. An electrode sealing assembly according to claim 4, wherein the non-electrically conductive fluid is one of the group consisting of air, nitrogen and carbon dioxide.

## Patentansprüche

1. Elektrodenabdichtanordnung (700), gestaltet zur Verwendung mit einem elektrochirurgischen Instrument zum Abdichten von Gewebe, umfassend:
ein erstes und ein zweites Klauenelement (710, 720), die aus einer ersten Position in einer beabstandeten Beziehung relativ zueinander in zumindest eine zweite Position beweglich sind, um Gewebe dazwischen zu ergreifen, wobei die Klauenelemente enthalten:
elektrisch leitende Abdichtplatten (712a, 712b), die in gegenüberliegender Beziehung zueinander angeordnet sind, wobei die elektrisch leitenden Abdichtplatten entsprechende Gewebe kontaktierende Oberflächen (727a, 727b) aufweisen, um Gewebe dazwischen zu ergreifen und abzudichten, wenn die Klauenelemente in der zweiten Position sind, wobei zumindest ein Klauenelement enthält:
eine thermoelektrische Kühlplatte (718, 728), wobei die thermoelektrische Kühlplatte erste und zweite elektrische Verbindungen (734a, 734b, 736a, 736b) enthält, die auf gegenüberliegenden Seiten der thermoelektrischen Kühlplatte angeordnet sind, wobei die erste Verbindung eingerichtet ist, um wahlweise ein erstes elektrisches Potential zu übertragen, und die zweite Verbindung eingerichtet ist, um wahlweise ein zweites elektrisches Potential zu übertragen, so dass Wärme, die durch die Abdichtplatten erzeugt wird, von dem Gewebe über die thermoelektrische Kühlplatte weg transportiert wird;
eine erste Oberfläche (730, 740) der thermoelektrischen Kühlplatte und eine äußere Oberfläche der Abdichtplatte mit einem dazwischen angeordneten thermisch leitenden, elektrisch isolierenden Material (780, 782), so dass die auf der Gewebe kontaktierenden Oberfläche der elektrisch leitenden Abdichtplatte erzeugte Wärme durch die Abdichtplatte und durch das thermisch leitende, elektrisch isolierende Material zu der ersten Oberfläche der thermoelektrischen Kühlplatte transportiert wird; **dadurch gekennzeichnet, dass**
das zumindest eine Klauenelement ferner einen Kühlkörper (818, 828) enthält, der in direktem Kontakt mit einer zweiten Oberfläche (732, 742) der thermoelektrischen Kühlplatte angeordnet ist, wobei der Kühlkörper eine Kühlmittelleitung (850, 852) enthält, die durch den Kühlkörper (818, 823) angeordnet ist.

2. Elektrodenabdichtanordnung nach Anspruch 1, wobei der Kühlkörper eingerichtet ist, um an einen zweiten Kühlkörper (1250) gekoppelt zu sein, um Wärme von dem zumindest einen der Klauenelemente zu transportieren.

3. Elektrodenabdichtanordnung nach Anspruch 1 oder 2, wobei die Kühlmittelleitung eingerichtet ist, um ein Kühlmittel aufzunehmen, um Wärme von der thermoelektrischen Kühlplatte zu transportieren.

4. Elektrodenabdichtanordnung nach Anspruch 3, wobei das Kühlmittel ein thermisch leitendes, elektrisch nichtleitendes Fluid ist.

5. Elektrodenabdichtanordnung nach Anspruch 4, wobei das elektrisch nichtleitende Fluid eins der Gruppe ist, die aus Luft, Stickstoff und Kohlendioxid besteht.

## Revendications

1. Ensemble de scellement à électrode (700) conçu pour l'utilisation avec un instrument électrochirurgical pour le scellement de tissu, comprenant:
des premier et deuxième éléments de mâchoire (710, 720) déplaçables d'une première position, en une relation espacée l'un de l'autre, à au moins une deuxième position pour saisir le tissu entre eux, les éléments de mâchoire incluant:
des plaques de scellement électriquement conductrices (712a, 712b) disposées d'une manière opposée l'une à l'autre, les plaques de scellement électriquement conductrices ayant des surfaces respectives venant en contact avec le tissu (727a, 727b) pour saisir et sceller le tissu entre elles lorsque les éléments de mâchoire se trouvent dans la deuxième position, au moins un élément de mâchoire incluant:
une plaque de refroidissement thermoélectrique (718, 728), ladite plaque de refroidissement thermoélectrique comprenant des première et deuxième connections électriques (734a, 734b, 736a, 736b) disposées sur des côtés opposés de la plaque de refroidissement thermoélectrique, ladite première connection étant configurée pour transmettre sélectivement un premier potentiel électrique, et ladite deuxième connection étant configurée pour transmettre sélectivement un deuxième potentiel électrique de sorte que la chaleur produite par les plaques de scellement est transférée au loin du tissu par la plaque de refroidissement thermoélectrique;
une première surface (730, 740) de la plaque de refroidissement thermoélectrique et une surface externe de la plaque de scellement ayant un matériau thermiquement conducteur, électriquement isolant (780, 782) disposé entre celles-ci de sorte que la chaleur produite sur la surface venant en contact avec le tissu de la plaque de scellement électriquement conductrice est transférée à travers la plaque de scellement et à travers le matériau thermiquement conducteur, électriquement isolant à la première surface de la plaque de refroidissement thermoélectrique;
**caractérisé en ce que** le au moins un élément de mâchoire comprend en outre un dissipateur de chaleur (818, 828) disposé en contact direct avec une deuxième surface (732, 742) de la plaque de refroidissement thermoélectrique, où le dissipateur de chaleur comprend une conduite d'agent de refroidissement (850, 852) disposée à travers le dissipateur de chaleur (818, 828).

2. Ensemble de scellement à électrode selon la revendication 1, dans lequel le dissipateur de chaleur est configuré pour être couplé à un deuxième dissipateur de chaleur (1250) pour transférer la chaleur du au moins un des éléments de mâchoire.

3. Ensemble de scellement à électrode selon la revendication 1 ou 2, dans lequel la conduite d'agent de refroidissement est configurée pour recevoir un agent de refroidissement afin de transférer la chaleur de la plaque de refroidissement thermoélectrique.

4. Ensemble de scellement à électrode selon la revendication 3, dans lequel l'agent de refroidissement est un fluide thermiquement conducteur, non-électriquement conducteur.

5. Ensemble de scellement à électrode selon la revendication 4, dans lequel le fluide non-électriquement conducteur est un du groupe consistant en air, azote et dioxyde de carbone.
